# EUROPEAN PATENT SPECIFICATION

(11) **EP 4 389 075 B1**
(45) Date of publication and mention of the grant of the patent: **11.02.2026**
(21) Application number: 23215820.4
(22) Date of filing: 12.12.2023
(51) Int. Cl.: A61F 2/26

(54) **AN ERECTILE DYSFUNCTION TREATMENT SYSTEM WITH A PENILE IMPLANT ASSEMBLY INCLUDING A REAR TIP EXTENDER ATTACHABLE TO A PENILE IMPLANT**
SYSTEM ZUR BEHANDLUNG VON EREKTILER DYSFUNKTION MIT EINER PENISIMPLANTATANORDNUNG MIT EINEM AN EINEM PENISIMPLANTAT ANBRINGBAREN HINTEREN SPITZENEXTENDER
SYSTÈME DE TRAITEMENT DE DYSFONCTIONNEMENT ÉRECTILE AVEC UN ENSEMBLE D'IMPLANT PÉNIEN COMPRENANT UN PROLONGATEUR DE POINTE ARRIÈRE POUVANT ÊTRE FIXÉ À UN IMPLANT PÉNIEN

(30) Priority: 20.12.2022 US 202263433771 P
(43) Date of publication of application: 26.06.2024
(73) Proprietor: Coloplast A/S, 3050 Humlebaek (DK)
(72) Inventor: Shouman, Khaled Said, Minneapolis, 55411 (US)

(56) References cited:
- EP-A1- 3 357 458
- EP-A2- 0 052 858
- WO-A1-2020/081366
- US-A1- 2003 220 539
- US-A1- 2012 157 764
- US-A1- 2020 289 269

## Description

### Background

An implanted penile prosthetic is an effective approach to relieving male erectile dysfunction.

A penile prosthesis (a device is a prosthesis prior to implant and a prosthetic after implantation) could be a malleable and non-inflatable device or an inflatable device. The malleable device has a flexible core that is bendable to various orientations, which for example allows the user to bend the penile implant to a desired erect shape or to a relaxed state. An inflatable penile prosthesis typically includes a pair of cylinders implantable in the penis, a reservoir implantable in the abdomen, and a pump implantable in the scrotum that is employed to move liquid from the reservoir into the cylinders. Pumping liquid from the reservoir to the cylinders inflates the penile implant to allow for penetrative intercourse.

Each of these penile prostheses has a portion that is implanted within the penis. The distal portion of the penile prosthesis is implanted within a dilated corpus cavemosum, and the proximal portion is implanted within a dilated crus penis. There is often a variation in the depth and width of the dilated crus penis, even within an individual patient. The surgeon desires to appropriately fit the proximal portion of the penile prosthesis within the crus and might desire to attach length-extenders to the implant to adjust for the depth of the crus penis. These length-adjusting accessories should be easy to attach yet resistant to detaching from the implant in the case of explant.

US2020/0289269 discloses a rear tip extender for a penile implant including a support segment and a receiving segment couplable to a distal end of the penile implant. EP 0 052 858 A2, US 2012/157764 A1, and EP 3 357 458 A1 disclose penile prostheses which can be extended based on the patient needs with different coupling features.

Surgeons and users would benefit from implantable penile prostheses with length-adjusting accessories that are easy to attach yet resistant to detaching if explanted.

### Brief Description of the Drawings

The accompanying drawings are included to provide a further understanding of embodiments and are incorporated in and constitute a part of this specification. The drawings illustrate embodiments and together with the description teach principles of this disclosure. Other embodiments and the intended advantages of embodiments will be readily appreciated as they become better understood by reference to the following detailed description. The elements of the drawings are not necessarily to scale relative to each other. Like reference numerals designate corresponding similar parts.
Fig. 1 is a perspective view of one embodiment of a penile implant assembly including a rear tip extender (RTE) attachable to a penile implant.
Fig. 2 is a perspective view of the RTE aligned for attachment to a proximal portion of the penile implant.
Fig. 3 is a perspective view of one embodiment of a key useful in coupling the RTE to the penile implant.
Fig. 4A is a side view and Fig. 4B is a cross-sectional view of the key configured to couple the RTE to the penile implant.
Fig. 5A is a side view of the RTE and Fig. 5B is a cross-sectional view of the RTE showing a slot and a cavity adapted to lock relative to the key shown in Fig. 3.
Fig. 6 is a cross-sectional view of the slot and the cavity shown in Fig. 5B.
Fig. 7 is a cross-sectional view of a lock having the key engaged with the slot and the cavity.
Fig. 8 is a cross-sectional view of the lock with the key inserted into the slot and the cavity.
Fig. 9 is a cross-sectional view of the lock with the key partially engaged with the distal RTE wall of the cavity.
Fig. 10 is a cross-sectional view of the key locked against the distal RTE wall of the cavity.
Fig. 11 is a schematic view of the assembly of Fig. 1 implanted in a patient.

### Summary

The invention is set out in the appended set of claims. An erectile dysfunction treatment system is disclosed as is a penile implant assembly. The erectile dysfunction treatment system includes the penile implant assembly, a pump, and a reservoir. The penile implant assembly includes a penile implant attachable to a rear tip extender (RTE). The RTE allows adjustment of an effective implanted length for the penile implant, where the disclosed RTE is easy to attach to the implant yet resistant to detaching from the implant if the assembly is explanted.

One embodiment includes a penile implant comprising a distal portion implantable into a corpus of an external portion of a penis and a proximal portion implantable into a crus of an internal portion of the penis, with the proximal portion of the penile implant comprising
a recess formed in a distal direction into the proximal portion,
a key located within the recess, with the key comprising a paddle connected to a post, with the post connected to the penile implant and extending longitudinally within the recess; and
a rear tip extender (RTE) attachable to the key, with the RTE forming an opening including a slot communicating with a cavity, with the cavity bounded by a proximal RTE wall and a distal RTE wall;
wherein, when the RTE is attached to the key, the post is received within the slot and the paddle is received within the cavity, and rotation of the RTE relative to the key captures the paddle between the proximal RTE wall and the distal RTE wall.

An aspect of the above embodiment includes wherein the post is connected to a base wall of the recess, and a length of the post measured from the base wall to the paddle is not greater than a distance of the distal RTE wall from a distal end of the RTE such that rotation of the RTE relative to the key forces the paddle against the distal RTE wall.

An aspect of the above embodiment includes wherein the distance of the distal RTE wall measured from the distal end of the RTE increases along an arc of the distal RTE wall such that rotation of the RTE relative to the key forces the paddle to pull the RTE toward the penile implant.

An aspect of the above embodiment includes wherein rotation of the RTE relative to the key drags the distal RTE wall against a distal face of the paddle to pull the RTE in a direction toward the penile implant.

An aspect of the above embodiment includes wherein an exterior wall of the penile implant forms a collar surrounding the recess, and the RTE comprises a flange insertable into the collar of the penile implant, and the rotation of the RTE relative to the key engages a thread flight on the collar with a complimentary thread flight on the flange.

An aspect of the above embodiment includes wherein a portion of the paddle extends longitudinally in the proximal direction a distance outside of the recess.

An aspect of the above embodiment includes wherein the paddle has a paddle width extending in a lateral direction, and the paddle width is wider than the post and the paddle and the post having an identical thickness.

An aspect of the above embodiment includes wherein a proximal end and lateral sides of the paddle have a clearance space relative to surfaces of the cavity when the paddle is captured between the proximal RTE wall and the distal RTE wall.

Another embodiment includes a penile implant assembly comprising:
a penile implant comprising a distal portion implantable into a corpus of an external portion of a penis and a proximal portion implantable in a crus of an internal portion of the penis; and
a rear tip extender (RTE) attachable to the proximal portion of the penile implant;
wherein the penile implant comprises a key extending longitudinally from the proximal portion of the penile implant, with the key comprising a paddle connected to a post, with the paddle having a paddle length measured between a proximal face opposite a distal face;
wherein the RTE forms an opening including a slot communicating with a cavity, with the cavity having a longitudinal boundary formed by a proximal RTE wall opposite a distal RTE wall;
wherein, when the key is inserted into the opening, the post is received within the slot and the paddle length is received within the cavity, and rotation of the RTE relative to the key moves the RTE toward the penile implant by forcing the distal RTE wall against the distal face of the paddle.

An aspect of the above embodiment includes wherein a length of the longitudinal boundary of the cavity is less than the paddle length such that the proximal face and the distal face of the paddle are press fit between the proximal RTE wall and the distal RTE wall, respectively, upon rotation of the RTE relative to the key.

An aspect of the above embodiment includes wherein an exterior wall of the penile implant forms a collar surrounding the key, and the RTE comprises a flange insertable into the collar of the penile implant, and the rotation of the RTE relative to the key engages a thread flight on the flange with a complimentary thread flight on the collar.

An aspect of the above embodiment includes wherein a distance of the distal RTE wall measured from the distal end of the RTE increases along an arc of the distal RTE wall such that rotation of the RTE relative to the key proportionally tightens the RTE against the penile implant.

Another embodiment includes an erectile dysfunction treatment system, the system comprising:
a pump and a reservoir attachable to a penile implant assembly, the penile implant assembly comprising:
a penile implant comprising a distal portion implantable into a corpus of an external portion of a penis and a proximal portion implantable in a crus of an internal portion of the penis; and
a rear tip extender (RTE) attachable to the proximal portion of the penile implant;
wherein an external wall of the proximal portion of the penile implant comprises collar surrounding a recess;
wherein the penile implant comprises a key, with the key comprising a paddle connected to a post that extends longitudinally from the recess, with the paddle having a paddle length measured between a proximal face and a distal face of the paddle and a paddle width that is larger than a width of the post;
wherein the RTE comprises a flange insertable into the recess and an opening formed in the flange including a slot communicating with a cavity, with the cavity bounded by a proximal RTE wall and a distal RTE wall;
wherein, when the flange is inserted into the collar and the key is inserted into the opening, the paddle is received within the cavity, and rotation of the flange within the collar compresses the distal RTE wall against the distal face of the paddle to draw the RTE toward the penile implant.

An aspect of the above embodiment includes wherein a surface of the distal RTE wall is not orthogonal relative to a distal end of the flange.

An aspect of the above embodiment includes wherein a distance of the distal RTE wall measured from a distal end of the flange increases along an arc of the distal RTE wall such that rotation of the RTE relative to the key forces the paddle to pull the RTE toward the penile implant.

An aspect of the above embodiment includes wherein rotation of the flange within the collar engages a thread flight on the collar with a complimentary thread flight on the flange.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in different orientations, the directional terminology is used for purposes of illustration and is in no way limiting.

The features of the various exemplary embodiments described in this application may be combined with each other, unless specifically noted otherwise.

The term "proximal" as employed in this application means that part that is oriented closest to a center of the human body.

The term "distal" as employed in this application means that part that is situated farthest away from the center of the human body. A distal end is the furthest endmost location of a distal portion of a thing being described, whereas a proximal end is the nearest endmost location of a proximal portion of the thing being described. As relates to penile implants, the proximal portion of the penile implant is implanted in the crus penis and the distal portion of the penile implant is implanted in the pendulous (external portion) of the penis.

The length and width of the penile prosthesis that is implanted in the distal portion of the penis is selected by the surgeon after the surgeon measures the length and girth of each dissected corpus cavernosum. Common sizes for the penile prosthesis implanted in each corpus cavernosum are characterized as "standard" or "narrow." The length of the prosthesis implanted in the crus penis may be adjusted by using one or more rear tip extenders that are attached to the proximal portion of the prosthesis.

Surgeons desire rear tip extenders that are easy to attach and resistant to detaching from the implant. We describe a lock feature between the rear tip extender (RTE) and the penile implant that allows the RTE to be effectively and robustly attached to the implant during the surgical procedure while also ensuring that the RTE is resistant to detaching from the implant if the surgeon determines it is desirable to explant the device. Explantation of the device allows the surgeon to replace the penile assembly or portions of the assembly, and it is desirable to remove the RTE (if employed) along with the implant. The lock feature we describe below durably connects the RTE to the implant for the duration of the use of the implant, including explantation, if directed by a surgeon.

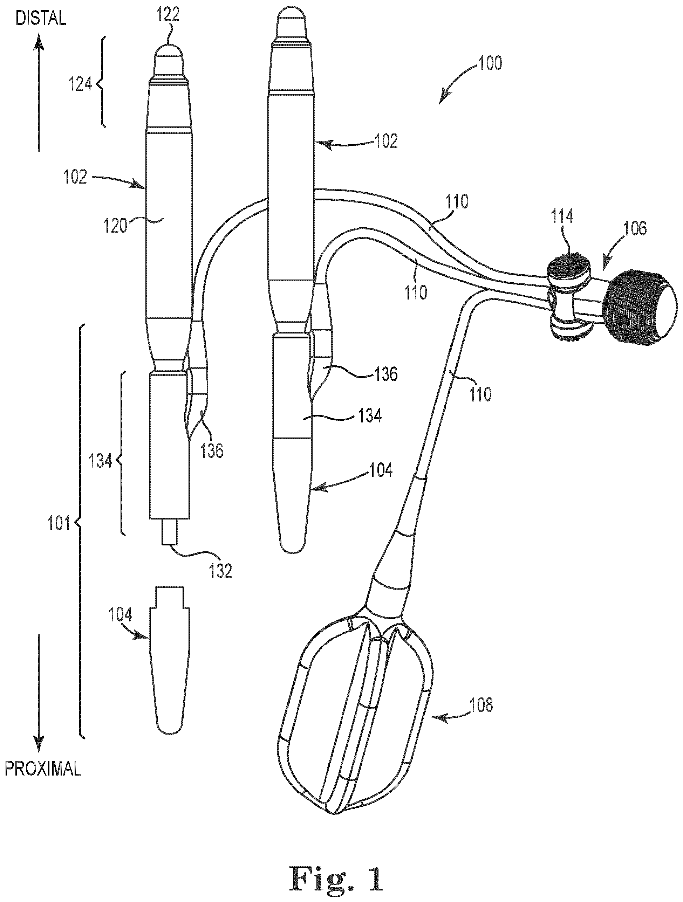

Fig. 1 is a perspective view of one embodiment of an erectile dysfunction treatment system 100. The system 100 is implantable into a body to provide a person suffering from erectile dysfunction with a penile erection.

The system 100 includes a penile implant assembly 101 attachable to a pump 106 and a reservoir 108.

The penile implant assembly 101 includes a penile implant 102 that is implantable into a corpus of the penis and a rear tip extender 104 (RTE 104) attachable to the penile implant 102. The natal male penis has two corpora, so an example of an effective system 100 includes two penile implants 102, one for each corpus of the penis. The penile implant 102 illustrated on the left-hand side of Fig. 1 has the RTE 104 positioned for attachment to the implant 102 and the penile implant 102 on the right-hand side of Fig. 1 has the RTE 104 already connected to the implant 102.

The pump 106 is attached or attachable between the penile implants 102 and the reservoir 108, for example with suitable tubing 110 connected between the pump 106 and the penile implants 102 and other like tubing 110 connected between the pump 106 and the reservoir 108.

The RTE 104 advantageously allows a surgeon to adjust the length of each of the implants 102 of the penile implant assembly 101. Some patients have a relatively deep crus penis compared to other patients, and some patients have a relatively shorter crus penis, which can arise when scar tissue forms within the crus penis. The RTE 104 allows the surgeon to customize a size of the penile implant assembly 101 for each patient. The RTE 104 is designed to allow the surgeon to act on his or her decision for the size of the implant while in the surgery room, which is to say that the RTE 104 is provided at varying lengths and attaches to the penile implants 102 quickly and durably. One RTE 104 is shown uncoupled to the penile implant 102 and one RTE 104 is shown coupled to the penile implant 102. Each RTE 104 may have a different length which allows the surgeon to individually adjust the length of each of the implants 102 of the penile implant assembly 101.

The directional naming convention employed in this description is that the center of the patient defines the proximal location and a direction away from the patient, for example toward the external penis, is the distal location. Thus, the RTE 104 is attachable to a proximal portion of each implant 102 and implanted proximally within the crus, and an opposite end of the implant 102 is implanted distally within the external penis.

The pump 106 is provided to move liquid out from the reservoir 108 and into the penile implants 102. One embodiment of the pump 106 includes a pump bulb, where squeezing of the pump bulb moves the liquid from the reservoir 108 into the implants 102. The displaced liquid remains under pressure within the implants 102 to provide the implants 102 with a rigidity sufficient for penetrative intercourse. A release pad 114 is provided on the pump 106 and pressing the release pad 114 displaces a valve stem inside the pump 106 to allow the pressurized liquid to return from the penile implants 102 back to the reservoir 108. One suitable pump 106 is available from Coloplast Corp., Minneapolis, Minnesota as part of an erectile dysfunction treatment product sold under the registered trademark TITAN penile prosthesis.

The reservoir 108 is sized to maintain a volume of liquid between about 50-300 ml. In one embodiment, the reservoir 108 is provided as a "cloverleaf" style of reservoir having multiple leaves that may be folded one against the other to compact the reservoir 108 for implantation into the abdomen of the user. One suitable reservoir 108 is sized to retain approximately 130 mL of liquid and is available from Coloplast Corp., Minneapolis, Minnesota.

The RTE 104 is suitable for use with a malleable (or, not inflatable) penile implant or with an inflatable penile implant. The penile implants 102 shown in Fig. 1 are inflatable penile implants, each of which is inflatable with liquid from the reservoir 108 to achieve a stiffness suitable for penetrative intercourse. Each of the penile implants 102 include an inflatable cylinder 120 extending from a distal end 122 of a distal portion 124 of the implant 102 to a rear portion of the implant 102. The rear portion of the implant 102 includes a proximal-most end 132 located on a proximal portion 134 of the penile implant 102. A connection tube 136 is connected to the proximal portion 134 of the penile implant 102, where the connection tube 136 is adapted to couple with the tubing 110 and provide a level of strain relief for the implants 102.

The inflatable cylinders 120 are fabricated from material configured to collapse when the cylinders 120 are deflated to provide the penis with a flaccid state and expand (like a balloon) when the cylinders 120 are inflated with liquid to provide the penis with an erection. As a point of reference, the cylinders 120 are illustrated in an inflated state. Suitable material for fabricating the cylinders 120 includes silicone, biocompatible polymers such as urethanes, blends of polymers with urethane, copolymers of urethane, or the like.

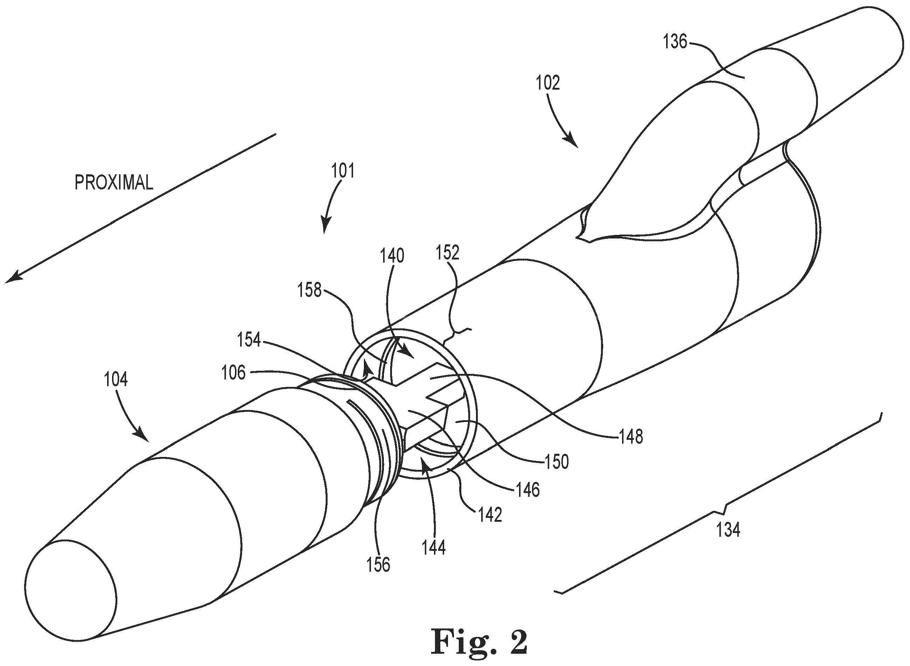

Fig. 2 is a perspective view of one embodiment of the penile implant 102 aligned for coupling with the RTE 104. The proximal portion 134 of the penile implant 102 includes a recess 140 formed in an end 142 of the proximal portion 134 of the implant 102 with a key 144 located within the recess 140. The key 144 includes a paddle 146 connected to a post 148, with the post 148 connected to a base wall 150 of the recess 140 and extending longitudinally in the proximal direction away from the recess 140. In one embodiment, a portion of the paddle 144 extends longitudinally a distance outside of the recess 140. Alternatively, it is acceptable to have the paddle 144 extending longitudinally and retained within a profile of the recess 140.

The key 144 is fixed to the implant 102 and provides a locking feature to ensure the RTE 104 remains engaged with the implant 102. When the RTE 104 is attached relative to the key 144 a keyed lock if formed that connects the RTE 104 to the implant 102 in a manner that resists separation or detachment of the RTE 104 from the implant 102. The positive connection of the RTE 104 to the implant 102 by the keyed lock feature is useful if the surgeon decides to explant the implant assembly 101.

In one approach, the RTE 104 is rotated or turned relative to the key 144 to durably engage the key 144 with the RTE 104. In one embodiment, an additional engagement feature is provided in the form of locking threads cooperatively formed between the implant 102 and the RTE 104. In one example, an exterior wall 152 of the penile implant 102 forms a collar 154 surrounding the recess 140, and the RTE 104 provides a flange 156 insertable into the collar 154 of the penile implant 102. When the key 144 is inserted into the RTE 104, rotation of the RTE 104 engages the RTE 104 with the key 144 and engages a thread flight 158 on the collar 154 with a complimentary thread flight 160 on the flange 156.

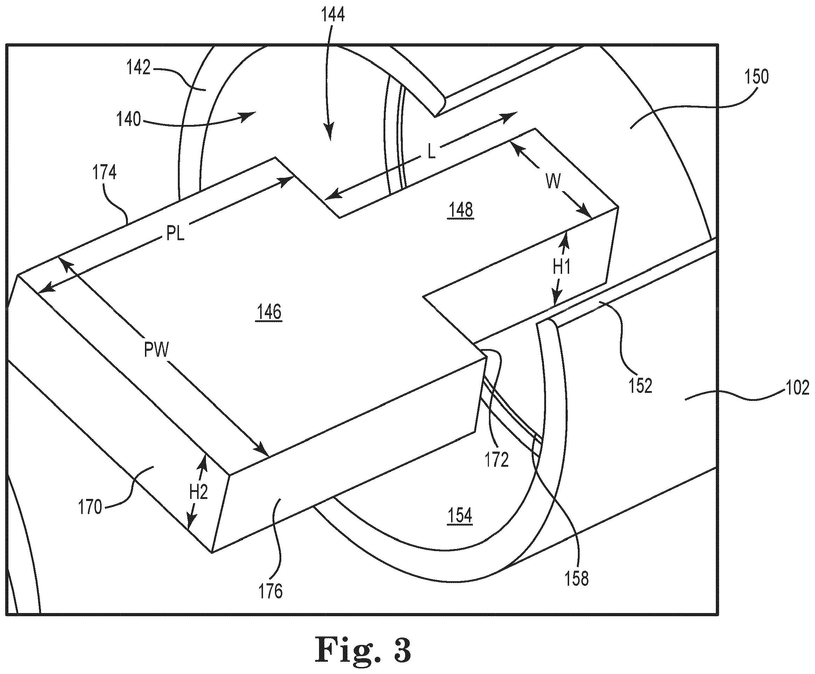

Fig. 3 is a perspective view of the key 144. The key 144 has the post 148 attached to the base wall 150 of the recess 140, where the post 148 is attached to the paddle 146. The post 148 has a length L extending from the base wall 150 to the paddle 146; a width W; and a height H1. The paddle 146 has a paddle length PL measured longitudinally between a proximal face 170 opposite a distal face 172; a paddle width PW extending in a lateral direction between opposing sides 174, 176 of the paddle 146; and a paddle height H2 separating opposing major faces of the paddle 146. In one embodiment, the key 144 is integrally formed where the paddle width PW is wider than the width W of the post 148 and the post height H1 is identical in thickness to the height H2 of the paddle 146.

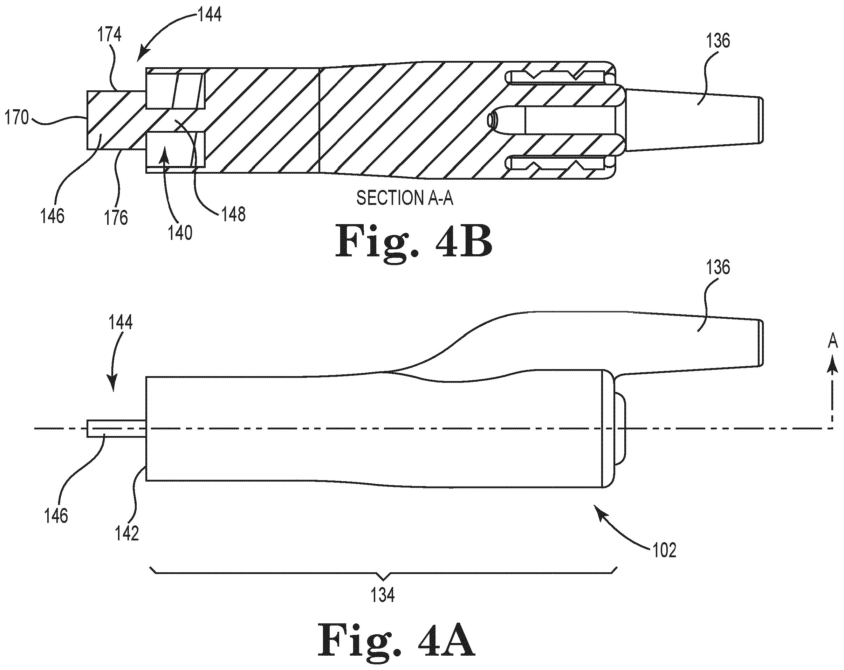

Fig. 4A is a side view and Fig. 4B is a cross-sectional view of the key 144 extending longitudinally from the proximal portion 134 of the penile implant 102. In this embodiment, the paddle 146 projects longitudinally entirely outside of the recess 140 formed in the proximal end 142 of the implant 102.

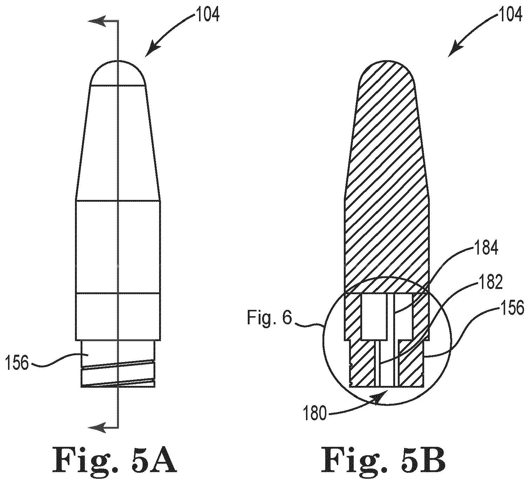

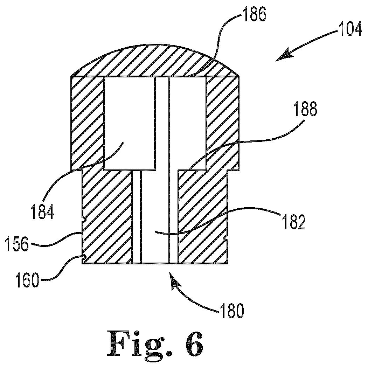

Fig. 5A is a side view of the RTE 104, Fig. 5B is a cross-sectional view of the RTE 104, and Fig. 6 is an enlarged cross-sectional view of a cavity formed in the RTE 104 that is adapted to lock with the key 144 shown in Fig. 3.

The RTE 104 forms an opening 180 including a slot 182 communicating with a cavity 184. The slot 182 provides an entry to the cavity 184, and the cavity 184 is bounded by a proximal RTE wall 186 and a distal RTE wall 188.

With reference to Fig. 3 and Fig. 6, the slot 182 and the cavity 184 are sized such that, when the RTE 104 is attached to the key 144, the post 148 is received within the slot 182 and the paddle 146 is received within the cavity 184, and rotation of the RTE 104 relative to the key 144 captures the paddle 146 between the proximal RTE wall 186 and the distal RTE wall 188.

In one embodiment, the paddle 146 and the cavity 184 are sized to allow a press fit where, when the RTE 104 is rotated relative to the key 144, an initial positive clearance between the distal face 172 of the paddle 146 and the distal RTE wall 188 diminishes to zero, which effectively applies a rearward locking force from the paddle 146 to the RTE 104 in a distal direction to secure the RTE 104 against the penile implant 102. Aspects of this beneficial feature may be obtained by providing the distal RTE wall 188 with a taper converging or leaning in the proximal direction that will cause the distal RTE wall 188 to drag along the distal face 172 of the paddle 146 as the RTE 104 is rotated.

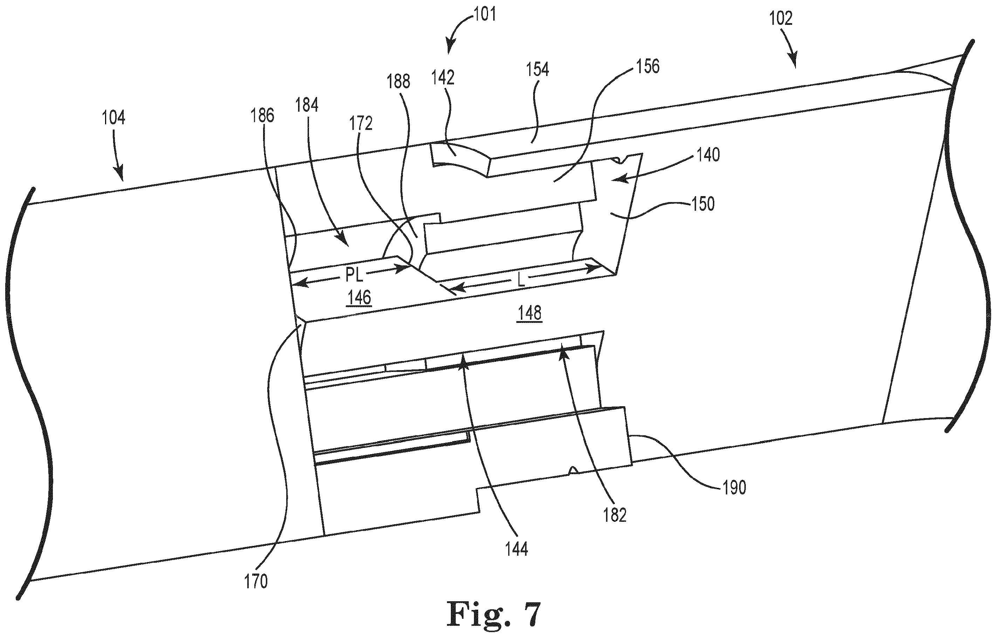

Fig. 7 is a cross-sectional view of a lock formed by the key 144 of the penile implant 102 engaged with the slot 182 and the cavity 184 of the RTE 104.

The key 144 has been inserted into the opening 180 and the flange 156 of the RTE 104 engages with the collar 154 of the penile implant 102. The post 148 is received within the slot 182 and the paddle 146 is received within the cavity 184. There is clearance between the post 148 and the slot 182 to allow movement of the RTE 104 relative to the key 144. There is an initial clearance between the proximal face 170 of the paddle 146 and the proximal RTE wall 186 and between the distal face 172 of the paddle 146 and the distal RTE wall 188, which allows the RTE 104 to move relative to the key 144.

To complete assembly of the RTE 104 onto the penile implant 102, the surgeon rotates the RTE 104 to engage the key 144 with the cavity 184. The initial positive clearance between the distal face 172 of the paddle 146 and the distal RTE wall 188 diminishes to zero during rotation of the RTE 104 to drive the RTE 104 in the distal direction to compress or retain the RTE 104 against the penile implant 102. The length PL of the paddle 146 is sized so the paddle 146 positively engages, for example by becoming frictionally squeezed, between the proximal RTE wall 186 and the distal RTE wall 188. The positive engagement of the paddle 146 of the implant 102 within the cavity 184 of the RTE 104 durably engages the RTE 104 to the implant 102 to ensure the two parts remain connected in case of a future possible explantation.

The post 148 is connected to a base wall 150 of the recess 140, and the length L of the post 148 measured from the base wall 150 to the paddle 146 is not greater than a distance of the distal RTE wall 188 to a distal end 190 of the RTE 104 such that rotation of the RTE 104 relative to the key 144 forces the paddle 146 against the distal RTE wall 188. In one embodiment, the length L of the post 148 measured from the base wall 150 to the paddle 146 is less than the distance of the distal RTE wall 188 to a distal end 190 of the RTE 104, which ensures compression of the RTE 104 distally into engagement with the penile implant 102.

In one embodiment, the distal end 190 of the RTE 104 is also the distal end of the flange 156, and a surface of the distal RTE wall 188 is not orthogonal to the distal end 190 of the flange 156. The distal RTE wall 188 is inclined or protrudes in the proximal direction. In this case, a distance measured from the distal end 190 of the flange 156 to a location on the distal RTE wall 188 increases along an arc of the distal RTE wall 188, for example along the arc that is traced by the paddle 146 as the RTE 104 is rotated. The non-orthogonal distal RTE wall 188 causes the rotation of the RTE 104 relative to the key 144 to force the paddle 146 to pull the RTE 104 toward the penile implant 102, or in the distal direction, as the inclined distal RTE wall 188 moves relative to the paddle 146. The non-orthogonal aspect of the distal RTE wall 188 in combination with the paddle 146 of the key 144 causes the RTE 104 to become tighter relative to the implant 102, which results in the RTE 104 staying on the implant 102 even during a possible explant procedure.

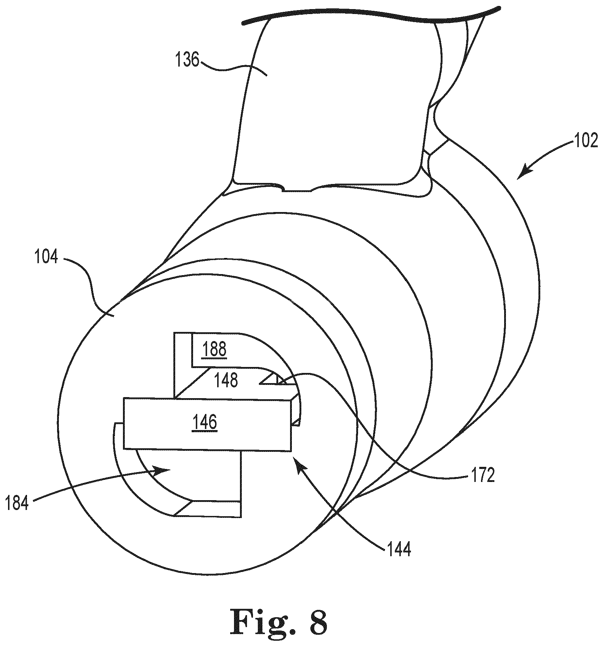

Fig. 8 is a cross-sectional view of the lock with the key 144 initially inserted into the slot 182 and the cavity 184 with clearance between the post 148 and the slot 182.

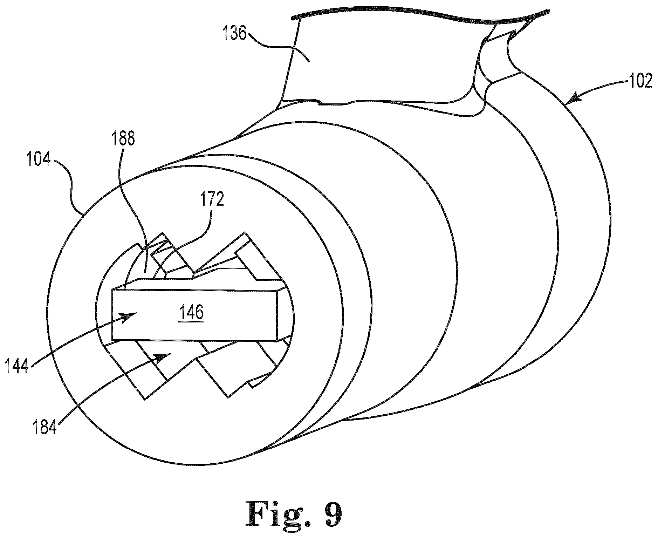

Fig. 9 is a cross-sectional view of the lock with the distal face 172 of the paddle 146 contacting and engaged with the distal RTE wall 186 of the cavity 184. In this orientation, the RTE 104 has been rotated by about 45 degrees relative to the penile implant 102.

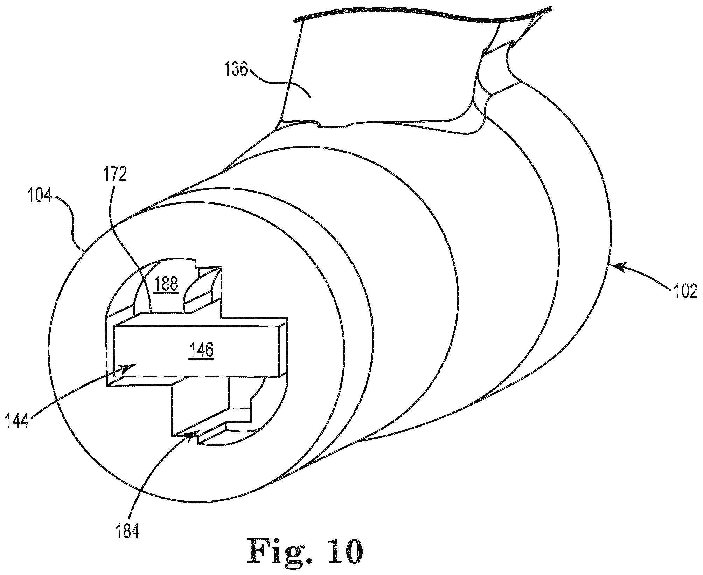

Fig. 10 is a cross-sectional view of the key 144 locked against the distal RTE wall 186 of the cavity 184. There is no clearance between the distal face 172 of the paddle 146 and the distal RTE wall 188 such that these two components touch. The distal face 172 of the paddle 146 is frictionally engaged with the distal RTE wall 188, which pulls the RTE 104 in the distal direction to compress or retain the RTE 104 against the penile implant 102.

A variety of RTEs 104 may be provided with the system 100 where each of the RTEs 104 may have a different length L and a different added effective length Le when attached to the implant 102. Surgeons have indicated a preference to have a selection of RTEs 104 available at the time of surgery and it is beneficial to provide RTEs 104 in increasing sizes of 0.5 cm increments (for example, a selection of RTEs with lengths L sized from 1.0 cm, 1.5 cm, 2.0 cm, 2.5 cm, to 3.0 cm, etc.). In one embodiment, the RTE 104 is a three-dimensional conical body and includes a tapered diameter that tapers from a larger diameter at a distal end to a smaller, rounded diameter at a proximal end. The taper may be linear or a combination of non-tapered and tapered sections. One suitable material for fabricating the RTE 104 is silicone, for example a liquid silicone rubber, of a durometer between 50A - 70A Shore hardness.

The surgeon usually measures the depth and width of the crus penis and the length of the distal (or external) penis before selecting a penile implant 102. If the surgeon determines that the length of the implant should be increased, the surgeon will select a suitably sized RTE 104 and connect it to the implant 102.

Suitable dimensions for the RTE 104 are provided as an example. One suitable RTE 104 provides an added effective length Le of 2.0 cm when attached to the implant 102. As noted above, a variety of RTEs 104 would typically be provided in a kit to accommodate adding an effective length Le (Fig. 3) to the penile implant in increments of 0.5 cm. The proximal end of the RTE 104 has a tip radius of 2.0 mm. The distal end of the RTE 104 has an outside diameter of about 10 mm and is selected to smoothly match a diameter of the implant 102 measured at the proximal portion 134. Thus, the RTE diameter measured at the distal end is selected to match the diameter of the proximal end portion 134 of the implant 102, which can vary depending on whether the implant 102 is selected to be of a "narrow" size or a wider "standard" size.

We have described a keyed lock useful in attaching a RTE to a penile implant. As described above, the key 144 is provided on the penile implant 102 and the complementary cavity 184 to receive the key 144 is provided in the RTE 104, where the cavity 184 includes the proximal RTE wall 186 and the distal RTE wall 188 that contact and engage the paddle 146 of the key during an attachment step. Alternatively, it is also acceptable to provide the key 144 projecting from the RTE component and to provide the complementary cavity to receive the key 144 in the penile implant 102 component.

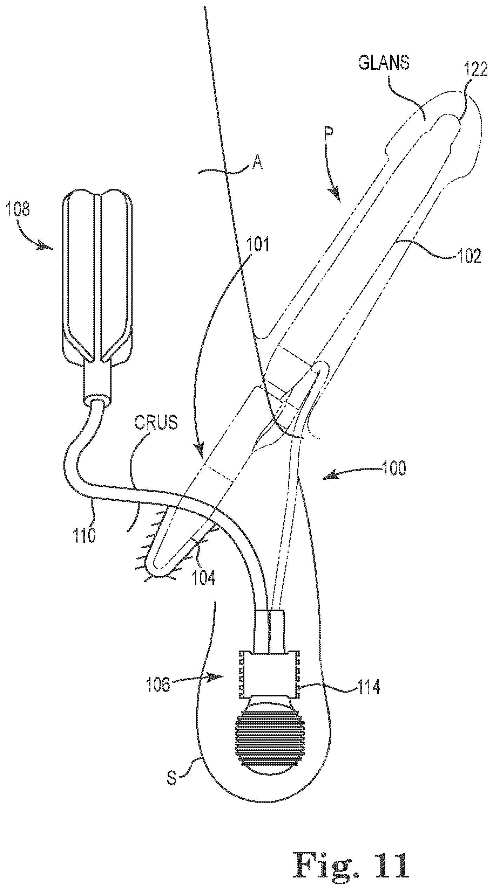

Fig. 11 is a schematic side view of the erectile dysfunction treatment system 100 implanted in a patient. One penile implant assembly 101 is shown having the penile implant 102 implanted in a corpus of the external penis and the RTE 104 implanted in the crus penis. The other penile implant assembly 101 is blocked from the side view.

The groin area of the patient is shaved, antiseptically cleaned, for example with a surgical solution, and draped with a sterile drape. A retraction device, such as a surgical retractor sold under the trademark Lone Star and available from Lone Star Medical Products of Stafford, TX, is placed around the penis P. Thereafter, the surgeon forms an incision to access the corpora cavernosa of the patient, where suitable examples of incisions include either an infrapubic incision or a scrotal incision. The infrapubic incision is initiated between the umbilicus and the penis (i.e., above the penis), whereas the transverse scrotal incision is made across an upper portion of the patient's scrotum S. As an example of the transverse scrotal approach, the surgeon forms a 2-3 cm transverse incision through the subcutaneous tissue of the median raphe of the upper scrotum S and dissects down through the Dartos fascia and Buck's fascia to expose the tunicae albuginea of the penis P. Thereafter, each corpus cavernosum is exposed in a corporotomy where a small (approximately 1.5 cm) incision is formed to allow the surgeon to access and subsequently dilate each corpus cavernosum. The surgeon typically will insert an instrument (such as a blunt-ended scissors or other elongated tool) to separate a portion of the spongiosum material to open a path that allows insertion of a device to measure the proximal and distal length of each corpus cavernosum. Thereafter, each corpus cavernosum is dilated distally with a suitable expanding tool to create a space for the penile implant 102. In one approach, the surgeon begins dilation of the penis by introducing an 8 mm dilator into the spongy tissue of the corpora and the crus with sequential progression to about a 14 mm dilator, each of which are introduced and pushed distally toward the glans penis and then proximally toward the crus of the penis, respectively. After suitable dilation of the space within the penis, the surgeon selects an implant 102 and adjusts the effective length by selecting an appropriately sized RTE 104.

The surgeon inserts and directs the distal end 122 of each of the penile implants 102 into the distal-most portion of each corpus cavernosum. The proximal end of the RTE 104 is implanted into each of the dilated proximal crus penis. The corporotomy is closed, and the remaining portions of the pump 106 and the reservoir 108 of the assembly 100 are implanted in the scrotum S and the abdomen A, respectively, of the patient.

The RTE 104 advantageously provides the surgeon with options in adjusting the effective length of the implant 102. The lock provided by the key 144 secures the RTE 104 to the implant 102 for the usable lifetime of the implant 102, even if explanted.

## Claims

1. A penile implant assembly (101) comprising:
a penile implant (102) comprising a distal portion (124) implantable into a corpus of an external portion of a penis and a proximal portion (134) implantable into a crus of an internal portion of the penis, the proximal portion (134) of the penile implant comprising:
a recess (140) formed in a distal direction into the proximal portion (134),
a key (144) located within the recess (140), with the key (144) comprising a paddle (146) connected to a post (148), with the post (148) connected to the penile implant (102) and extending longitudinally within the recess (140); and
a rear tip extender (RTE) (104) attachable to the key (144), with the RTE (104) forming an opening (180) including a slot (182) communicating with a cavity (184), with the cavity (184) bounded by a proximal RTE wall (186) and a distal RTE wall (188);
wherein, when the RTE (104) is attached to the key (144), the post (148) is received within the slot (182) and the paddle (146) is received within the cavity (184), and rotation of the RTE (104) relative to the key (144) captures the paddle (146) between the proximal RTE wall (186) and the distal RTE wall (188),
**characterized in that**
a portion of the paddle (146) extends longitudinally in the proximal direction a distance outside of the recess (140).

2. The penile implant assembly of claim 1, wherein the post (148) is connected to a base wall (150) of the recess (140), and a length of the post (148) measured from the base wall (150) to the paddle (146) is not greater than a distance of the distal RTE wall (188) from a distal end (190) of the RTE (104) such that rotation of the RTE (104) relative to the key (144) forces the paddle (146) against the distal RTE wall (188).

3. The penile implant assembly of claim 2, wherein the distance of the distal RTE wall (188) measured from the distal end (190) of the RTE (104) increases along an arc of the distal RTE wall (188) such that rotation of the RTE (104) relative to the key (144) forces the paddle (146) to pull the RTE (104) toward the penile implant (102).

4. The penile implant assembly of claim 1, wherein rotation of the RTE (104) relative to the key (144) drags the distal RTE wall (188) against a distal face (172) of the paddle (146) to pull the RTE (104) in a direction toward the penile implant (102).

5. The penile implant assembly of claim 1, wherein an exterior wall (152) of the penile implant (102) forms a collar (154) surrounding the recess (140), and the RTE (104) comprises a flange (156) insertable into the collar (154) of the penile implant (102), and the rotation of the RTE (104) relative to the key (144) engages a thread flight (158) on the collar (154) with a complimentary thread flight (160) on the flange (156).

6. The penile implant assembly of claim 1, wherein the paddle (146) has a paddle width extending in a lateral direction, and the paddle width is wider than the post (148) and the paddle (146) and the post (148) having an identical thickness.

7. The penile implant assembly of claim 1, wherein a proximal end and lateral sides of the paddle (146) have a clearance space relative to surfaces of the cavity (184) when the paddle (146) is captured between the proximal RTE wall (186) and the distal RTE wall (188).

## Patentansprüche

1. Penisimplantatanordnung (101), umfassend:
ein Penisimplantat (102), das einen distalen Abschnitt (124) umfasst, der in einen Korpus eines äußeren Abschnitts eines Penis implantierbar ist, und einen proximalen Abschnitt (134), der in einen Schenkel eines inneren Abschnitts des Penis implantierbar ist, wobei der proximale Abschnitt (134) des Penisimplantats umfasst:
eine Aussparung (140), die in einer distalen Richtung in dem proximalen Abschnitt (134) gebildet ist,
einen Schlüssel (144), der sich in der Aussparung (140) befindet, wobei der Schlüssel (144) ein Paddel (146) umfasst, das mit einem Pfosten (148) verbunden ist, wobei der Pfosten (148) mit dem Penisimplantat (102) verbunden ist und sich in Längsrichtung in der Aussparung (140) erstreckt; und
eine Verlängerung der hinteren Spitze (RTE) (104), die an dem Schlüssel (144) anbringbar ist, wobei die RTE (104) eine Öffnung (180) bildet, die einen Schlitz (182) aufweist, der mit einem Hohlraum (184) kommuniziert, wobei der Hohlraum (184) von einer proximalen RTE-Wand (186) und einer distalen RTE-Wand (188) begrenzt wird;
wobei, wenn die RTE (104) an dem Schlüssel (144) angebracht ist, der Pfosten (148) in dem Schlitz (182) aufgenommen wird und das Paddel (146) in dem Hohlraum (184) aufgenommen wird und die Drehung der RTE (104) bezogen auf den Schlüssel (144) das Paddel (146) zwischen der proximalen RTE-Wand (186) und der distalen RTE-Wand (188) einfängt,
**dadurch gekennzeichnet, dass**
sich ein Abschnitt des Paddels (146) eine Distanz außerhalb der Aussparung (140) in Längsrichtung in die proximale Richtung erstreckt.

2. Penisimplantatanordnung nach Anspruch 1, wobei der Pfosten (148) mit einer Basiswand (150) der Aussparung (140) verbunden ist, und eine von der Basiswand (150) bis zu dem Paddel (146) gemessene Länge des Pfostens (148) nicht größer ist als eine Distanz der distalen RTE-Wand (188) von einem distalen Ende (190) der RTE (104), so dass die Drehung der RTE (104) bezogen auf den Schlüssel (144) das Paddel (146) gegen die distale RTE-Wand (188) drängt.

3. Penisimplantatanordnung nach Anspruch 2, wobei der von dem distalen Ende (190) der RTE (104) gemessene Abstand der distalen RTW-Wand (188) entlang eines Bogens der distalen RTE-Wand (188) zunimmt, so dass die Drehung der RTE (104) bezogen auf den Schlüssel (144) das Paddel (146) zwingt, die RTE (104) in Richtung des Penisimplantats (102) zu ziehen.

4. Penisimplantatanordnung nach Anspruch 1, wobei die Drehung der RTE (104) bezogen auf den Schlüssel (144) die distale RTE-Wand (188) gegen eine distale Fläche (172) des Paddels (146) zieht, um die RTE (104) in eine Richtung hin zum Penisimplantat (102) zu ziehen.

5. Penisimplantatanordnung nach Anspruch 1, wobei die Außenwand (152) des Penisimplantats (102) einen Kragen (154) bildet, der die Aussparung (140) umgibt, und die RTE (104) einen Flansch (156) umfasst, der in den Kragen (154) des Penisimplantats (102) einsetzbar ist, und die Drehung der RTE (104) bezogen auf den Schlüssel (144) bewirkt, dass ein Gewindegang (158) an dem Kragen (154) einen komplementären Gewindegang (160) an dem Flansch (156) in Eingriff nimmt.

6. Penisimplantatanordnung nach Anspruch 1, wobei das Paddel (146) eine Paddelbreite hat, die sich in eine laterale Richtung erstreckt, und die Paddelbreite breiter ist als der Pfosten (148) und das Paddel (146) und der Pfosten (148) eine identische Dicke haben.

7. Penisimplantatanordnung nach Anspruch 1, wobei ein proximales Ende und die lateralen Seiten des Paddels (146) einen Zwischenraum bezogen auf die Oberflächen des Hohlraums (184) haben, wenn das Paddel (146) zwischen der proximalen RTE-Wand (186) und der distalen RTE-Wand (188) eingefangen ist.

## Revendications

1. Ensemble d'implant pénien (101) comprenant :
un implant pénien (102) comprenant une partie distale (124) implantable dans un corps d'une partie externe d'un pénis et une partie proximale (134) implantable dans un pilier d'une partie interne du pénis, la partie proximale (134) de l'implant pénien comprenant :
un évidement (140) formé dans une direction distale dans la partie proximale (134),
une clé (144) située à l'intérieur de l'évidement (140), la clé (144) comprenant une palette (146) reliée à un montant (148), le montant (148) étant relié à l'implant pénien (102) et s'étendant longitudinalement à l'intérieur de l'évidement (140) ; et
un prolongateur de pointe arrière (RTE) (104) apte à être fixé à la clé (144), le RTE (104) formant une ouverture (180) incluant une fente (182) communiquant avec une cavité (184), la cavité (184) étant délimitée par une paroi proximale du RTE (186) et une paroi distale du RTE (188) ;
le montant (148) étant reçu dans la fente (182) et la palette (146) étant reçue dans la cavité (184) lorsque le RTE (104) est fixé à la clé (144), et la rotation du RTE (104) par rapport à la clé (144) capturant la palette (146) entre la paroi proximale du RTE (186) et la paroi distale du RTE (188),
**caractérisé en ce que**
une partie de la palette (146) s'étend longitudinalement dans la direction proximale sur une distance à l'extérieur de l'évidement (140).

2. Ensemble d'implant pénien selon la revendication 1, le montant (148) étant relié à une paroi de base (150) de l'évidement (140), et une longueur du montant (148) mesurée depuis la paroi de base (150) jusqu'à la palette (146) n'étant pas supérieure à une distance de la paroi distale du RTE (188) par rapport à une extrémité distale (190) du RTE (104), de sorte que la rotation du RTE (104) par rapport à la clé (144) force la palette (146) contre la paroi distale du RTE (188).

3. Ensemble d'implant pénien selon la revendication 2, la distance de la paroi distale du RTE (188) mesurée à partir de l'extrémité distale (190) du RTE (104) augmentant le long d'un arc de la paroi distale du RTE (188) de telle sorte que la rotation du RTE (104) par rapport à la clé (144) force la palette (146) à tirer le RTE (104) en direction de l'implant pénien (102).

4. Ensemble d'implant pénien selon la revendication 1, la rotation du RTE (104) par rapport à la clé (144) tirant la paroi distale du RTE (188) contre une face distale (172) de la palette (146) pour tirer le RTE (104) en direction de l'implant pénien (102).

5. Ensemble d'implant pénien selon la revendication 1, une paroi extérieure (152) de l'implant pénien (102) formant un collier (154) entourant l'évidement (140), et le RTE (104) comprenant une bride (156) apte à être insérée dans le collier (154) de l'implant pénien (102), et la rotation du RTE (104) par rapport à la clé (144) mettant en prise un filet (158) sur le collier (154) avec un filet complémentaire (160) sur la bride (156).

6. Ensemble d'implant pénien selon la revendication 1, la palette (146) ayant une largeur de palette s'étendant dans une direction latérale, et la largeur de palette étant supérieure à celle du montant (148), et la palette (146) et le montant (148) ayant une épaisseur identique.

7. Ensemble d'implant pénien selon la revendication 1, une extrémité proximale et des côtés latéraux de la palette (146) présentant un espace de dégagement par rapport à des surfaces de la cavité (184) lorsque la palette (146) est capturée entre la paroi proximale du RTE (186) et la paroi distale du RTE (188).
